(19)

Europäisches
Patentamt
European
Patent Office
Office européen
des brevets

(11) **EP 2 836 958 B1**

(12) **EUROPEAN PATENT SPECIFICATION**

(45) Date of publication and mention
of the grant of the patent:
**10.06.2020 Bulletin 2020/24**

(21) Application number: **13720102.6**

(22) Date of filing: **10.04.2013**

(51) Int Cl.:
***G06K 9/00*** *(2006.01)*          ***G06K 9/62*** *(2006.01)*

(86) International application number:
**PCT/NL2013/050260**

(87) International publication number:
**WO 2013/154425 (17.10.2013 Gazette 2013/42)**

(54) **METHOD FOR CLASSIFICATION OF A SAMPLE ON THE BASIS OF SPECTRAL DATA AND CORRESPONDING DATA STORAGE MEDIUM AND SYSTEM**

VERFAHREN ZUR KLASSIFIKATION EINER PROBE AUF GRUNDLAGE VON SPEKTRALDATEN SOWIE ENTSPRECHENDES DATENTRÄGER UND SYSTEM

PROCÉDÉ DE CLASSIFICATION D'UN ÉCHANTILLON SUR LA BASE DE DONNÉES SPECTRALES, SUPPORT DE STOCKAGE DE DONNÉES ET SYSTÈME CORRESPONDANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.04.2012 NL 2008620
15.06.2012 NL 2009015**

(43) Date of publication of application:
**18.02.2015 Bulletin 2015/08**

(73) Proprietor: **BiosparQ B.V.
2333 BD Leiden (NL)**

(72) Inventors:
 • **PARCHEN, René Raymond
  2333 BD Leiden (NL)**
 • **VAN WUIJCKHUIJSE, Arjan Laurens
  2333 BD Leiden (NL)**
 • **BOS, Adrianus
  2333 BD Leiden (NL)**

(74) Representative: **Vernout, Robert
Arnold & Siedsma
Bezuidenhoutseweg 57
2594 AC The Hague (NL)**

(56) References cited:
 **EP-A1- 2 157 599          WO-A1-2012/044170
 DE-A1-102005 002 672    US-A1- 2007 184 455
 US-A1- 2012 065 899**

 • **MERAY L: "Deconvolution of nuclear spectra of low counts", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NETHERLANDS, vol. A353, no. 1, 30 December 1994 (1994-12-30), pages 272-275, XP004005874, ISSN: 0168-9002, DOI: 10.1016/0168-9002(94)91655-1**
 • **SHARAABI Y ET AL: "Two-state analysis of single-molecule Raman spectra of crystal violet", CHEMICAL PHYSICS, NORTH-HOLLAND, NL, vol. 318, no. 1-2, 15 November 2005 (2005-11-15), pages 44-49, XP027604023, ISSN: 0301-0104 [retrieved on 2005-11-15]**
 • **DIECKMANN R ET AL: "Rapid identification and characterization of Vibrio species using whole-cell MALDI-TOF mass spectrometry", JOURNAL OF APPLIED MICROBIOLOGY 2010 BLACKWELL PUBLISHING LTD GBR, vol. 109, no. 1, July 2010 (2010-07), pages 199-211, XP002691231, ISSN: 1364-5072**

**(Cont. next page)**

- **H. OBERREUTER ET AL.: "Quantification of micro-organisms in binary mixed populations by Fourier transform infrared (FT-IR) spectroscopy", LETTERS IN APPLIED MICROBIOLOGY , vol. 30, no. 1 January 2000 (2000-01), pages 85-89, XP002691232, Wiley Online Library DOI: 10.1046/j.1472-765x.2000.00694.x Retrieved from the Internet: URL:http://onlinelibrary.wiley.com/doi/10. 1046/j.1472-765x.2000.00694.x/pdf [retrieved on 2013-01-28]**

- **TSUYOSHI SONEHARA ET AL: "Prism-Based Spectral Imaging of Single-Molecule Fluorescence from Gold-Nanoparticle/Fluorophore Complex", JOURNAL OF FLUORESCENCE, KLUWER ACADEMIC PUBLISHERS-PLENUM PUBLISHERS, NE, vol. 21, no. 4, 8 March 2011 (2011-03-08), pages 1805-1811, XP019930842, ISSN: 1573-4994, DOI: 10.1007/S10895-011-0875-6**

**Description**

The invention relates to a method for classification of a sample in one of at least two classes on the basis of spectral data. US 2007/0184455 A1 discloses a method for the determination of samples having a large number of identical particles, such as molecules. For example, the spectral data comprise a Raman spectrum, a near-infrared spectrum, a FT-IR spectrum, a frequency spectrum, a MALDI MS spectrum or a MALDI TOF-MS spectrum. The spectra obtained using one of these techniques can be related to the constituents of the sample, i.e. to the molecules present in the sample.

However, irrespective of the technique used for obtaining the spectra, variations occur between spectra of samples belonging to the same class. These variations can be attributed to stochastic effects. These stochastic effects can be related to the measurement technique or to the sample under investigation, or to both.

Therefore, conventional methods of analyzing spectral data require that the spectra to be classified are obtained from samples having a large number of particles, such as molecules. By requiring a large number of particles, the features in spectra due to stochastic processes will be suppressed compared to the features caused by stable processes. However, this renders the conventional methods unsuitable for analyzing samples with a small number of particles. When the stochastic effects are largely due to the measurement technique itself, conventional methods require that the spectra to be classified are so called sum spectra, also known as cumulative spectra, obtained by adding a large number of spectra of the same sample. With this approach the stochastic processes due to the measurement technique will be suppressed in the sum spectrum. However, this renders conventional methods unsuitable for classifying samples when no sum spectra can be obtained.

The above problems will be illustrated for the exemplary case wherein the spectral data is obtained using mass spectrometry (MS).

Traditional MS spectra, and Time of Flight (TOF) mass spectra in particular, exhibit large ion intensities at masses that correspond to the mass of highly abundant ions in the ion plume formed by ionisation. Generally, these highly abundant ions result from highly abundant molecules present in the analyte.

However, since there is a large variability in the ease with which molecules are ionised, the presence of highly abundant molecules will not automatically generate a large ion intensity.

Furthermore, the efficiency of ionisation depends on a large number of factors that may vary strongly from ionisation event to ionisation event. Examples are the amount of analyte present, ratio of matrix and analyte, the crystal structure of the matrix, and the intensity of the laser beam at the location of the matrix crystal and analyte. Thus, the ion intensity produced by single ionisation events will vary considerably.

On top of that, during ionisation the molecules under consideration may disintegrate into smaller fragments, some molecules may obtain a double or triple charge, molecules may cluster in the plume, etc. Hence, apart from high ion intensities occurring at masses that correspond to the masses of the analyte molecules, a single ionisation event spectrum will exhibit peaks at location not correlated with the mass of the analyte molecules. However, since the process of disintegration, charging and clustering is highly stochastic in nature, the position of the non-correlated peaks will vary strongly from ionisation event to ionisation event.

To produce robust and stable spectra, in traditional classification methods for MALDI MS a large number of single-ionisation events, recorded at different locations on the target-plate spot are summed.

Thus, the ion intensity caused by the stochastic processes during ionisation will be suppressed compared to the ion intensity caused by stable processes. Stable processes will generate narrow regions of high intensity in the summed spectra (the peaks), while the processes with a more stochastic character will generate a broad low-intensity response in the summed spectra (the base line).

The peaks that appear in a summed spectrum can be related to easily (efficiently) ionisable, abundant molecules in the analyte. Generally these peaks are used for the classification of the spectra, and are thus considered as the characterizing features of the spectra.

Since the resulting spectrum stems from a large number of single-ionisation events, recorded at different locations on the target-plate spot, these conventional MALDI spectra are the sum of the spectra of many particles of the sample. For example, in the case the sample comprises micro-organisms, conventional methods rely on spectra obtained from many micro-organisms.

Similar problems arise when applying conventional methods on single-particle spectra, such as single-aerosol-particle spectra. In conventional methods, to generate robust and stable spectra, a large number of single-aerosol-particle spectra obtained from micro-organisms that originate from a single isolate are combined into a sum spectrum. This sum spectrum is characterised by a finite number of distinct spectral features (peaks), superimposed on a smooth base line.

The individual single-particle spectra that build the accumulated spectrum are characterized by a large number of peaks and the absence of a baseline. Some peaks occur on locations that do coincide with the location of the peaks in the accumulated spectrum, other peaks occur on locations that do not. Furthermore, it appears that the single-particle spectra exhibit a large shot-to-shot variation.

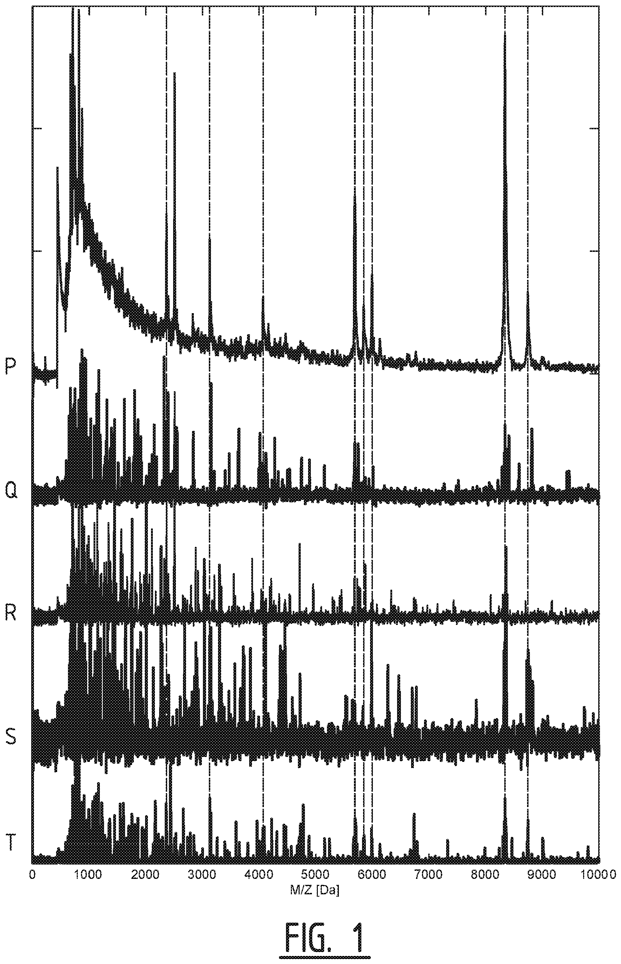

Figure 1 shows an example of spectrum (P) accumulated from a large number of single-particle spectra obtained from samples originating from a single isolate, together with a few typical examples (Q, R, S, T) of the underlying single-particle spectra.

Owing to the large shot-to-shot variation and the limited correlation between the single-particle peak locations and the accumulated spectrum peak locations, no deterministic relation exists between the amplitude of the spectrum at a certain location (mass over charge) and the presence of an ion-forming substance with the associated mass.

The above example illustrates the problems of conventional methods of analyzing spectral data. These methods are incapable of dealing with single particle spectra directly, since they do not take the above shot-to-shot variations into account. Furthermore, they are incapable of dealing with variations due to the measurement technique, such as the variations due to the ionisation in MALDI MS as described above.

A goal of the invention is to provide an improved method for classification of a sample in one of at least two classes on the basis of spectral data, which is effective and robust when spectra belonging to the same class exhibit variations.

This goal is achieved by the method in accordance with claim 1.

Steps a)-c) of claim 1 describe the creation of a reference library. Steps d) and e) describe comparing a sample to this reference library.

In step b) the value of the same at least one quantity is determined for each of the reference spectra. For example, a mathematical operation is performed on the spectral data to obtain this value. In other words, step b) describes obtaining a score (the value) by applying a predetermined function or operation on each of the reference spectra.

The result of step b) is that each of the reference spectra has associated with it at least one value, each value corresponding to a predetermined quantity, i.e. a predetermined operation on the spectrum. Next, for each set of reference spectra a probability is associated to different values of the at least one quantity on the basis of the values determined for the spectra in that set. This can be thought of as constructing a probability density function (PDF). For example, the PDF is a discrete probability density function, which may for example be represented as an array or histogram.

The end result of steps a)-c) is that each set of reference spectra has associated with it for each of the at least one quantities a PDF of the value of the respective quantity. For example, in the case of MS spectral data, one of the quantities selected is the intensity of the spectrum, also known as spectrum amplitude, at M/Z = 1000 Dalton. In this example, for each set of reference spectra a PDF is created for the intensity at M/Z = 1000 Dalton. It is noted that the PDFs will in general be very different for the different sets of reference spectra, as they correspond to different classes.

In step d) a spectrum of the sample is obtained, and also for this spectrum the value of the at least one quantity is determined. On the basis of the previously constructed PDFs the probability that the sample belongs to each one of the reference classes is calculated in step e).

Since the method according to the invention uses a PDF of a value (score) of a quantity (related to a predetermined operation / function) for each class, the method takes into account variations in the spectra of the same class. In particular, it is noted that both the presence and the absence of a feature, as represented by the value of the at least one quantity, is taken into account in the method according to the present invention.

A further advantage of the method according to the invention is that the end result in step e) is a probability. Since a probability is a normalized quantity, i.e. a probability is a value between 0 and 1, the probability that the sample spectrum belongs to a first reference class can be compared to the probability that the spectrum belongs to the second class. This is an advantage over conventional methods wherein a score is calculated which is not normalized.

Moreover, the invention enables defining a criterion for accepting or rejecting a classification. In general, the spectrum will be assigned to the class for which the probability that the sample spectrum belongs to that class is the highest. However, this probability may be relatively low, for example 0.2. The invention enables defining a threshold representing a minimum value for an acceptable classification. Sample spectra for which the maximum probability for all classes is below the threshold are not classified, i.e. they are classified in a class unknown. Such a procedure is not possible when a non-normalized score is used as in conventional methods, since the threshold in that case will be arbitrary.

As pointed out above, in particular in MALDI MS spectrum and MALDI TOF-MS spectra the variation between spectra belonging to the same class of particles is considerable. Therefore, the method according to the invention is in particular advantageous for these measurement techniques.

In a preferred embodiment of the present invention the reference spectra and spectrum obtained from the sample are spectra of single particles.

This represents the extreme case of a small number of particles. As noted above, the method according to the invention is able to account for the variation in spectra belonging to the same class which will be in particular present in spectra of single particles.

As mentioned above, the method according to the invention can be applied both for single particles and for two or more particles and even for many particles.

Preferably, the method comprises obtaining single particle spectra from a sample using MALDI MS, preferably MALDI TOF-MS. This enables the analysis of mixtures of particles. Instead of analyzing a spectrum obtained from many of the particles of the mixture, i.e. a composite spectrum, spectra are obtained for single particles of the mixture and the individual particles are classified on the basis of the single particle spectra.

For example, MALDI mass spectra, such as MALDI TOF MS spectra, are obtained of single particles by partitioning the sample into droplets containing at the most one particle. From these droplets aerosols are formed which are subsequently ionized and analysed using MALDI MS. Reference is made to WO 2010/021548, which describes a system and method to generate droplets containing at most one cell for subsequent analysis by MALDI MS. By using such a device, the spectra of individual cells of the mixtures are obtained. These spectra can be classified using the method according to the invention, to obtain a classification of the components of the sample.

The sample is a biological sample comprising micro-organisms. In particular, the reference spectra and the spectrum obtained from the sample are spectra of single particles of a biological sample. The variation of spectra of biological samples belonging to the same class is in particular pronounced, such that the method of the invention is in particular advantageous for biological samples. These variations stem for example from the life cycle of organisms and the differences between individuals of organisms of the same class, e.g. species.

The biological sample comprises micro-organisms and classification comprises the classification of micro-organisms.

The reference spectra and the spectrum obtained from the sample are spectra of single micro-organisms. This enables the analysis of mixtures of micro-organisms. For example, a mixture of micro-organisms is diluted and subsequently partitioned into droplets containing at most one particle, and subsequently spectra are obtained from the droplets using MALDI MS, such as described above.

In a preferred embodiment according to the invention the at least one quantity is selected on the basis of a characteristic special feature of a reference class.

For example, PDFs are obtained in steps a) to c) for classes A, B and C for the value of a quantity which relates to a characteristic spectral feature of class A. For example, the spectrum of class A shows a peak at location X as characteristic spectral feature. Correspondingly, the intensity of the spectrum at location X is selected as the value (score). This intensity at location X is calculated for all class A, class B and class C. Since a peak at location X is characteristic for class A, the PDF obtained for class A will show a high probability for high intensities at X. In general, class B and class C will show lower probabilities for high intensities at location X. So by selecting a characteristic spectral feature of a certain class as one of the at least one quantity, the method is able to determine if a spectrum belongs to this class on the basis of the presence or absence of the characteristic spectral feature.

Preferably, more than one quantity is selected on the basis of preferably more than one characteristic spectral feature of preferably more than one reference class. For example, for the classes A, B and C scores $I_1$ and $I_2$ are calculated which relate to two different spectral features of reference class A. In another example scores $I_1$, $I_2$ and $I_3$ are calculated, wherein $I_1$ relates to a spectral feature of class A, $I_2$ relates to a spectral feature of class B and $I_3$ relates to a spectral feature of class C.

Preferably, the characteristic spectral feature is determined on the basis of the cumulative spectrum of the reference class. The cumulative spectrum of the reference class can for example be obtained by summing the spectra of individual reference samples belonging to the same class. As noted above, these cumulative spectra will show features which are characteristic for a given class, although they may not be present in each individual spectra of the class. It is noted that the quantity can be determined using the sets of reference spectra as described in step a), by adding the reference spectra of each set and determining the characteristic spectral features of the sum spectra.

Preferably, the characteristic spectral features comprise a peak in the cumulative spectrum of the reference class. For example, a peak finding algorithm is applied to the sum spectrum to find the locations of characteristic peaks. The quantities selected are then the intensities at these peak locations or at a predetermined interval comprising these locations.

In a preferred embodiment of the invention the method comprises calculating, for a sample spectrum for which a value $I_i$ is obtained related to a spectral feature quantity $Qi$, the probability $P(A^j/I_i)$ that the sample belongs to the reference class $A^j$ given the value $I_i$ according to:

$$P\left(A^j\big|I_i\right) = \frac{P\left(I_i\big|A^j\right)P\left(A^j\right)}{P(I_i|A^j)P(A^j) + \sum_{k\neq j} P(I_i|A^k)P(A^k)}$$

wherein $P(I_i|A^j)$ is the probability associated with the value $I_i$ for the reference class $A^j$, and $P(I_i|A^k)$ with $k \neq j$ is the probability associated with the value $I_i$ for at least one reference class different from the reference class $A^j$.

The features $Q_i$ preferably are related to characteristic features of a particular class. For example, quantities $Q_1$, $Q_2$ are related to a feature of class $A^1$ and quantities $Q_3$, $Q_4$ and $Q_5$ are related to a feature of class $A^2$.

Note that in case a quantity $Q_i$ is related to a specific class $A^j$, this quantity may also be used to obtain a probability for a different class $A^{k \neq j}$. Illustrated with the example above, the value $I_2$ of quantity $Q_2$ is a measure for a characteristic feature of class $A^1$, however, it can be used to calculate $P(A^2|I_1)$, i.e. the probability that the spectrum belongs to class $A^2$ given the measurement of $I_1$.

When the classes are very distinct, this will result in a very low probability. However, such a calculation may become more important for classes of which the features are more similar.

In step c) of the method, the PDF has been calculated for a value $I_i$ for each reference class $A^j$. Using this PDF the probability of the value obtained for the sample, $P(I_i|A^j)$ is calculated. This probability represents the probability that the sample would give rise to the measured value $I_i$ if the sample belonged to reference class $A^j$. According to Bayes theorem this can be correlated to the probability that, given the measurement of the value $I_i$, the sample belongs the class $A^j$.

As indicated in the formula, this calculation also requires the summation of $P(I_i|A^k)P(A^k)$ over all other reference classes. Again, the value of $P(I_i|A^k)$ for each $k$ is obtained using the PDFs obtained in step c) of the method.

Preferably, the probabilities $P(A^j)$ are assumed to be equal for all $j$ (including $j = k$). It can be seen that the terms $P(A^j)$ and $P(A^k)$ then drop out of the equation. This approach is justified if we assume that the probability that the sample spectrum belongs to a reference class is equal for each reference class of the library. This approach is in particular useful when no prior knowledge is available about the content of the sample. If, however, knowledge is available on the basis of which the a priori probability of a sample belonging to a certain class is higher or lower than the other classes, different values for $P(A^j)$ can be used according to the invention.

In a preferred embodiment according to the invention step b) comprises determining for each of the reference spectra the value of the same at least two quantities related to a spectral feature and step e) comprises combining the probabilities obtained for all quantities to an overall probability that the sample belongs to the respective class.

If only one quantity is used in classifying the spectra using the method of the invention as described above, a single probability will be obtained for each reference class, corresponding to that one quantity. However, if more than one quantity is determined for the spectra, i.e. at least two different quantities, then for each reference spectra a number of probabilities is obtained, corresponding to each quantity. According to this embodiment of the invention these probabilities are combined to an overall probability, for example by a logical OR and/or AND. This translates in the summation and addition of the individual probabilities for the individual quantities.

This can be represented as a function F ($P(A^j|I_1)$, $P(A^j|I_2)$, ... , $P(A^j|I_n)$) for each of the reference classes $A^j$.

For the functions F two extreme forms can be formulated:

- all n features present in class $A^j$

$$\mathrm{F}\left(P\left(A^j \mid I_1\right), P\left(A^j \mid I_2\right), P\left(A^j \mid I_3\right), \dots, P\left(A^j \mid I_n\right)\right) = \prod_{i=1}^{i=n} P\left(A^j \mid I_i\right)$$

- any of the n features present in class $A^j$

$$\mathrm{F}\left(P\left(A^j \mid I_1\right), P\left(A^j \mid I_2\right), P\left(A^j \mid I_3\right), \dots, P\left(A^j \mid I_n\right)\right) = 1 - \prod_{i=1}^{i=n} P\left(\neg A^j \mid I_i\right),$$

where

$$P\left(\neg A^j \mid I_i\right) = \frac{P(I_i \mid \neg A^j)P\left(\neg A^j\right)}{P(I_i \mid A^j)P\left(A^j\right) + P(I_i \mid \neg A^j)P\left(\neg A^j\right)}.$$

Apart from the two above extreme forms, any other multivariate (self-learning) classification methods, such as Principal Component Analyses or Support Vector Machines can be used.

We note that the expression for $P(\neg A^j|I_i)$ can be obtained in a similar manner as described above for $P(A^j|I_i)$. In this case the PDFs of $P(\neg A^j|I_i)$ are obtained in steps a) - c) from the sets of reference classes. In fact, a single PDF is obtained on the basis of the values $I_i$ for all reference spectra not belonging to class $A^j$, i.e. all reference classes $A^k$ with $k \neq j$. For example, when three reference classes A, B and C are selected, the PDF for calculating $P(\neg C|I_i)$ is obtained on the basis of the values obtained for spectra of reference classes A and B.

In a preferred embodiment according to the present invention the value relates to the intensity at at least one predetermined spectral value or within a predetermined range of the spectral values.

For example, the value is a scalar which is equal to the intensity at a predetermined spectral value, i.e. the amplitude at a given location along the X-axis of the spectrum. In another example, the value is equal to the sum of the intensities of a predetermined range of spectral values.

For example, in a mass spectrum, the quantity is the peak intensity at a predetermined mass to charge ratio, or at a predetermined range of mass to charge ratios.

Preferably, the predetermined spectral value or the predetermined spectral range is selected on the basis of a characteristic spectral feature of a reference class. For example, the spectral value or the range of spectral values is selected as the value or range wherein the sum spectrum of a reference class shows a peak in the spectrum.

In a preferred embodiment the value obtained is normalized.

Normalization corrects for the variation in intensity of different spectra, for example due to the variation in total ion yield in MS spectra. Such variation may occur for example due to stochastic effects or due to drift of the measurement system. Furthermore, when using two or more measuring systems to obtain the spectral data for the method, differences in intensities are expected for spectra obtained by different systems.

Therefore, normalization leads to a more robust algorithm. For example, the value is normalized by dividing it by the sum of the intensities over the whole or part of the spectrum. The normalized spectra are then used to obtain the PDF according to step c) of the method. Also the value obtained from the sample spectrum is normalized to compare with the PDFs of each reference class for the corresponding quantity.

In a further preferred embodiment the value is determined by multiplying the respective spectrum over a predetermined range of spectral values with a weighting function.

For example, if the spectrum is represented as a vector, i.e. an array of a predetermined number of scalars representing the amplitude of the spectrum, the weighting function takes the form of a weighting vector. Multiplication of the weighting function with the spectrum in that case corresponds to taking the dot product of the weighting vector and the spectrum vector.

In practice, certain regions of the spectra are more relevant for a given quantity than others. This can be accounted for by multiplying with a weighting function. For example, the entire spectrum is multiplied by a weighting function. In most cases however, only a certain region of interest of the spectrum is selected and multiplied by a weighting function. We note that this is equivalent to multiplying the entire spectrum with a weighting function wherein the weighting function has a value of 0 outside the region of interest.

Preferably, the method comprises both normalizing the value and multiplication with a weighting function. We note that these steps may be combined by choosing an appropriate weighting function.

In a further preferred embodiment the weighting function is based on a cumulative spectrum of spectra of the same class.

As described above the weighting function gives more weight to important parts of the spectrum. What parts of the spectrum actually constitutes the important parts can be based on a cumulative spectrum the respective class. For example, a peak finding algorithm is applied to the cumulative spectrum to find the location of a peak in the sum spectrum and the weighting function is selected such that it puts more weight on the intensities at the location at and/or around the peak with respect to other parts of the spectra. For example, a peak is detected in the cumulative spectrum and the width of the peak is determined. The weighting function is then selected as a copy of the spectrum, wherein the value of the weighting function outside the peak width are set to zero. Optionally, the weighting function is also corrected for the baseline of the cumulative spectrum. Preferably, the weighting function is normalized such that the sum of its values is equal to 1, or the area under the spectrum curve is equal to 1.

Inherently, the weighting function of the above example will put most weight at the maximum of the peak and less weight at intensities far from the peak.

In a further preferred embodiment according to the invention, the value relates to the ratio between intensities at at least two predetermined spectral values or within at least two predetermined spectral ranges.

This has the advantage that the value is sensitive for a correlation between certain regions of the spectrum.

In an alternative embodiment the value relates to the location of a peak within a predetermined range of spectral values.

As mentioned above, the value can be a scalar value. However, in a preferred embodiment according to the invention the value is a vector. In this case the probability associated with the vector will be a multivariable probability density function $P(I_1, I_2, ..|A^j)$. For example, the elements of the vector are related to different quantities, i.e. the values are related to different spectral features. In the extreme case only one vector is calculated, wherein the elements of the vector correspond to all selected spectral features. In this case combining the probabilities of the different quantities using a logical AND and/or OR, e.g. combining these probabilities using the function F(...) as described above, is not necessary.

In a preferred embodiment according to the present invention the steps d) and e) are performed for a first set of reference classes and subsequently for a second set of reference classes, wherein the second set is selected on the basis of the classification of the sample in one of the reference classes of the first set.

This defines a hierarchy of reference libraries. The sample is first classified in a main class (the first set of reference classes) and subsequently classified in a subclass of the main reference class (the second set of reference classes). This is computationally less intensive than classifying using a reference library comprising all the lowest level classes. Therefore, the method according to the invention is efficient.

Furthermore, for each classification step, the sample is compared to a reference library containing relatively few reference classes. Therefore, the sets of reference classes can be selected such that the reference classes of each set show little overlap and the quality of classification increases.

Preferably, the first set and second set are selected on the basis of a biological classification hierarchy.

For example, when the sample comprises a biological sample, use can be made of a biological classification hierarchy for classification of the sample. For example, the choice of classification sets is based on order, family, genus, species or strain of micro-organism.

In a preferred embodiment, step d) comprises obtaining at least two spectra from the sample and determining the value of the same at least one quantity of these at least two spectra and step e) comprises combining the sample spectra classified in the same class into a cumulative spectrum and comparing this cumulative spectrum to a cumulative reference spectrum obtained by combining the reference spectra of the respective class.

This step provides an optional final check of the classification. Multiple spectra are obtained from the sample. These sample spectra are classified as described above. Subsequently, the spectra which are classified in the same class are combined, e.g. added, to obtain a cumulative sample spectrum. This cumulative sample spectrum is compared to the cumulative spectrum of reference spectra of the respective class.

We note that a sample may comprise a mixture of components, e.g. a mixture of different micro-organisms. Therefore, the spectra of such a mixture may be classified in different classes. In that case the spectra are combined per class.

For example, a sample comprises particles A and B. From the sample, 100 single-particle spectra are obtained. Using the method of the invention, 89 of the spectra are classified as belonging to class A and 11 are classified as belonging to class B. Subsequently a cumulative sample spectrum for class A is obtained by combining the respective 89 sample spectra. Also, for class B a cumulative sample spectrum is obtained using the respective 11 spectra. The cumulative sample spectra for class A and class B are then compared to the cumulative reference spectra for class A and class B. This provides a final check.

Preferably, the comparison of the cumulative sample spectrum and the cumulative reference spectrum puts more weight on quantities different from the at least one quantity of step b). In other words, the final check puts more weight on spectral features which have not been used to classify the sample spectra. Preferably, only these features are considered, i.e. the at least one quantity of step b) is not used in the comparison.

This ensures that the final check is largely or completely independent of the classification steps.

Preferably, the method is performed using a first set of reference classes and subsequently using a second set of reference classes, wherein the second set is selected on the basis of the comparison between the cumulative sample spectrum and the cumulative reference spectrum.

A hierarchical classification procedure is defined. The sample is first classified using a first library of reference classes. The classification is verified using the cumulative sample spectrum and the cumulative reference spectrum. If this comparison leads to the conclusion that the sample may be further classified in subclasses, the sample is subsequently compared to a second library.

For example, the comparison reveals that additional peaks are present in the cumulative spectrum of the sample as compared to the cumulative reference spectrum. This indicates that the sample may contain a mixture of micro-organisms, that belong to different classes. Therefore, the sample is subsequently compared to a second set of reference classes.

The invention further relates to a computer program which, when executed on a computer, executes the steps of one of the methods described above and a data storage medium comprising such a computer program.

Moreover, the invention relates to a system for classifying a sample on the basis of spectral data, comprising:

- means arranged to obtain a spectrum from a sample;
- analyzing means arranged to perform a method according to the invention as described above.

The same advantages and effects as described for the method for classifying a sample on the basis of spectral data apply to the method for creating a database, the method for using the database, the computer program, the storage medium and the system according to the invention. In particular, the features described in relation to the method for classifying a sample on the basis of spectral data can be combined with the method for creating a database, the method for using the database, the computer program, the storage medium and the system according to the invention.

In a preferred embodiment of the system according to the invention the system comprises means arranged to obtain a single-particle spectrum from a sample. For example, the system comprises means arranged to create droplets of the sample, such that the droplets comprises at most one particle, and the means arranged to obtain a spectrum are arranged to obtain a spectrum from the individual droplets.

Further advantages, features and details of the invention are elucidated on the basis of preferred embodiments thereof, wherein reference is made to the accompanying figures, in which:

- figure 1 shows a spectrum (P) accumulated from a large number of single-particle spectra obtained from samples originating from a single isolate, and a few typical examples (Q, R, S, T) of the underlying single-particle spectra;
- figure 2 shows a schematic overview of a system according to the invention including its subsystems;
- figure 3 shows an example of a peak shape graphically, for a raw spectrum (left graph) and a normalized and baseline corrected peak shape (right graph);
- figure 4 shows an example of the PDF of the score for a spectral feature for a particle collection (species A) that contains the feature-inducing substance ($P(I/A)$) and a collection (species B) that does not contain this substance ($P(I|\neg A)$) respectively;
- figure 5 shows the probability that the feature-inducing substance is present/absent as a function of the single-ionization event spectrum feature intensity, based on the probability density function presented in figure 4;
- figure 6 shows the reference for the species A containing particles, compiled by accumulating the single-particle spectra used to estimate the PDFs of the peaks at M/Z = 5689 and M/Z = 8339, indicated by vertical lines;
- figure 7 shows the reference for the species B containing particles, compiled by accumulating the single-particle spectra used to estimate the PDFs of the peaks at M/Z = 2187 and M/Z = 3558, indicated the by vertical lines;
- figure 8 shows a sequence of single-ionization event spectra recorded from a sample containing a mixture of two organisms (species A and species B);
- figure 9 shows the accumulated spectrum of the mixture of single-particle spectra (top), the single-particle spectra assigned to the species A class (middle) and the single-particle spectra assigned to the species B class (bottom);
- figure 10 shows a comparison between the accumulated spectrum of particles assigned to the species A class and the accumulated spectra of particles that stem from the species A isolate;
- figure 11 shows a comparison between the accumulated spectrum of particles assigned to the species B class and the accumulated spectra of particles that stem from the species B isolate;
- figure 12 shows an example of a clustergram of a collection of 95 *Staphylococcus aureus* strain based on the MALDI mass spectra recorded from these strains;
- figure 13 shows a hierarchical classification scheme according to the invention.

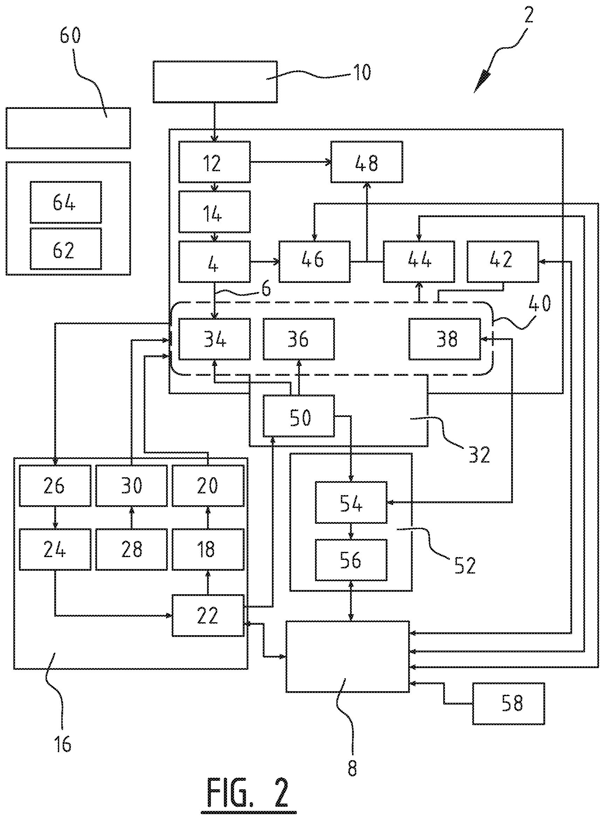

In an exemplary embodiment, a system 2 (figure 2) according to the invention is arranged to create spectra from aerosols for the detection of biological material such as bacteria in the air using MALDI TOF MS. The difference between this system and a classical MALDI instrument is the inlet which is an Aerosol Beam Generator 4, 12, 14 and the sample preparation, where the matrix is added inline. It is constructed in such a way that individual airborne particles enter the system in an Aerosol Beam 6. This opens the possibility to analyze individual particles such as bacteria, viruses or other biological material of a certain size in a mixture of aerosol. For example, medical application of the system is possible. The System Controller sub-system 8 is used to set, control, measure, log and monitor parameters from the sub-systems.

For the preparation of aerosols of the sample system 2 further comprises a sample preparation unit 10 inter alia for dilution of the sample, a first stage 12, aerodynamic lenses 14 and an assembly of nozzles and skimmers 4.

Laser subsystem 16 comprises ionization laser 18 and ionization optics 20, controlled by trigger electronics 22. Trigger electronics 22 is connected to system controller 8 and to photomultiplier tube detector 24 which is provided with photomultiplier optics 26. Furthermore a detection laser 28 and detection optics 30 are provided in subsystem 16.

Mass spectrometry subsystem 32 comprises ion source 34 which is the location where the aerosols are ionized using the laser bundle from laser 18. MS subsystem 32 further comprises an ion deflector 36 and an MS detector 38. The ion source 34, ion deflector 36 and MS detector 38 are contained in a recipient and flight tube 40. This tube is held vacuum by means of pressure gauges 42, an assembly of turbo-molecular pumps 44, 46 and backing pump 48. The ion source 34 and ion deflector 36 are connected to ion source control 50, which is connected to trigger electronics 22 and signal processing and data management subsystem 52.

Subsystem 52 comprises a digitizer 54 connected to MS detector 38. Via data management module 56 digitizer 54 is connected to the system controller 8. The system controller 8 is connected to analyzer 58.

The system 2 further comprises a power supply unit 60. Although no connections are drawn in the figure, this unit provides the power for the different components. Furthermore, a system rack 62 and an air / water cooling 64 are provided for housing system 2.

Using aerosolized bacteria and protein particles a proof of principle was realized on system 2. The method steps according to the invention are in this exemplary embodiment performed using analyzer 58.

**Establishing the presence of substances in single-particles**

Owing to the highly stochastic nature of single-ionisation event spectra in MS, the single particle spectrum intensity, *I*, at a location that corresponds to the mass of an analyte molecule, can only be considered as a measure for the probability, *P(A/I)*, that this analyte molecule, *A,* is present in the particle.

Quantifying this probability relies on Bayes' theorem and the probability density function, *P(I/A)*, that specifies the probability of the feature intensity *I*, given the presence of analyte molecule *A*:

$$P(A \mid I) = \frac{P(I \mid A)P(A)}{P(I \mid A)P(A) + P(I \mid \neg A)P(\neg A)},$$

where

- *P(A)* is the fraction of particles that contain substance *A;*
- *P(I/¬A)* is the probability density function for the intensity I for particles that do not contain substance *A;* and
- *P(¬A)* the fraction of particles that do not contain substance *A.*

Hence, provided that the functions *P(I/A)* and *P(I/¬A)* are known, the probability that the substance *A* is present in the particle can be derived from the measured intensity of the spectral feature that results from the presence of substance *A.*

Therefore, the reference information needed to classify single-particle spectra must contain the PDFs for all expected features.

**Establishing the origin of a single-cell based on its single-particle mass spectrum**

Micro-organisms such as bacteria are characterized by the presence of many substances that yield MALDI-MS distinguishable features. Depending on the organism (bacterium, virus, etc) and the state of the organism (vegetative cell, spore), this number may vary between as little as 5 to as many as 50 or even more.

Some of these features are representative for the genus to which the organism at hand belongs, some of them for the species and some of them for the strain.

For each feature, $Q_i$, a probability density function $P(I_i/A^j)$ is defined representing the probability of measuring an intensity $I_i$ for feature $Q_i$ of a spectrum of a sample comprising substance $A^j$.

Furthermore, a pdf $P(I_i/\neg A^j)$ is defined representing the probability of measuring a value $I_i$ for feature $Q_i$ when the sample does not comprise substance $A^j$.

These two pdf's are combined with $P(A^j)$ and $P(\neg A^j)$ in accordance with Bayes' theorem to obtain the probability $P(A^j/I_i)$ that the feature-inducing substance $A^j$ is present, given a measured feature intensity $I_i$.

It is noted that in this example, reference is made to a 'substance A$^j$' instead of a 'class A$^j$' as described above. In fact, this example represents the extreme case wherein a class A$^j$ comprises a single substance. For example, the substance comprises a particular protein.

To estimate the probability that a single-cell MALDI-MS spectrum stems from an organism belonging to a genus, species or strain, the probabilities for the individual features must be combined into a probability for the combination of features that are representative for a genus, species, or strain is present in the spectrum, using a function $F(P(A^j/I_1),$ $P(A^j/I_2), P(A^j/I_3), ..., P(A^j/I_n))$: $P(A^j|I_1,I_2,I_3,...,I_n) = F(P(A^j|I_1),P(A^j|I_2),P(A^j|I_3),...,P(A^j|I_n))$ giving the probability of a substance A$^j$ being present in the sample.

Furthermore, the probabilities for each substance A$^j$ can optionally be combined. For example, the probabilities for each protein of a set of proteins are combined to obtain an overall probability for a given micro-organism.

However, not all features that can be established in a spectrum that is accumulated from a large number of single-cell spectra, need to be present in every single-cell spectrum, see figure 1.

For example, during the different phases of their life cycle (just before fission, just after fission, etc), the cell may express different proteins. Hence, while the accumulated spectrum exhibits all ionizable proteins produced by the organisms during all phases of their life cycle, the spectrum of an individual organism can only exhibit those proteins that are expressed produced during the particular phase of particular cell that is analysed.

Strictly spoken, when the knowledge about the dependency of the protein expression on the phase in the life cycle is available, it could be possible to formulate the function *F(...)*. Alas, in general this information is not available.

However, as indicated above, apart from differences induced by differences in the life phase, there are many more causes for variability between single-cell spectra. Hence, even when the above information would be available, the function *F(...)* based on it, will be an estimate at best.

Neglecting the possibility of information on the relation between the life phase and the single-cell protein expression, two extreme forms of the function $F(...)$ can be formulated:

- all features present

$$F\left(P\left(A^j \mid I_1\right), P\left(A^j \mid I_2\right), P\left(A^j \mid I_3\right), \ldots, P\left(A^j \mid I_n\right)\right) = \prod_{i=1}^{i=n} P\left(A^j \mid I_i\right)$$

- any feature present

$$F\left(P\left(A^j \mid I_1\right), P\left(A^j \mid I_2\right), P\left(A^j \mid I_3\right), \ldots, P\left(A^j \mid I_n\right)\right) = 1 - \prod_{i=1}^{i=n} P\left(\neg A^j \mid I_i\right),$$

where

$$P\left(\neg A^j \mid I_i\right) = \frac{P(I_i \mid \neg A^j) P\left(\neg A^j\right)}{P(I_i \mid A^j) P\left(A^j\right) + P(I_i \mid \neg A^j) P\left(\neg A^j\right)}.$$

**Determining the probability density functions P(I|A) and P(I|¬A)**

**Introduction**

The PDFs for $P(I|A^j)$ and $P(I|\neg A^j)$ can be approximated by evaluation of a sufficiently large set of reference particles that do contain substance $A^j$ and do not contain substance $A^j$ respectively.

To determine $P(I_j|A^3)$ for all features $Q_i$, a set of single-particle spectra must be used of particles that are known to contain the substance $A^j$ that generates feature $Q_i$.

The features $Q_i$ are characterized by an expectation value of the intensity, at the masses associated with this feature, that is larger than the expectation value at adjacent mass regions. Thus, if a large number of single-particle spectra is summed, the accumulated spectrum will feature a peak (or combination of peaks in case of a polymer).

The PDF for a feature, characterised by a peak in the accumulated spectrum, can simply be estimated by recording the amplitude at the associated mass for each single particle spectrum binning these amplitudes into discrete amplitude containers and dividing the score in each bin by the total number of single-particle spectra recorded.

Given a sufficiently large number of single-particle spectra and a sufficiently fine bin distribution, this will yield an adequate estimate of the probability-density function.

**Extracting features**

For any real mass spectrometer the peaks in the accumulated spectrum will have a finite width. Hence, when recording the single-particle amplitude for each feature, one must allow for a finite width of the mass interval where the feature may occur.

Here, the shape of the peak in the accumulated spectrum is used to take the effect of a finite peak width into account. For this purpose, the shape of the accumulated spectrum for the interval of masses where the peak may occur is copied, corrected for the base-line amplitude at the edge of the interval and normalized such that AUC $=\int I(MZ)dMZ=1$.

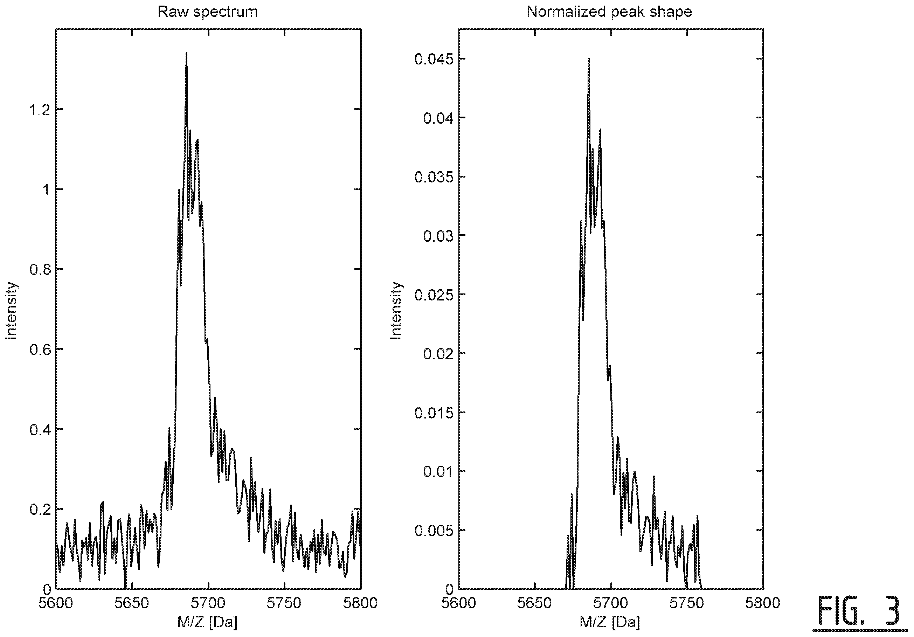

Figure 3 shows an example of a peak shape graphically.

Thus a feature shape function $S_{feature}$ can be defined such that:

$$S_{\text{feature}}(MZ) = \begin{cases} 0 & \text{for } MZ < MZ_{\min} \vee MZ > MZ_{\max} \\ F\left(I_{\text{spectrum}}(MZ)\right) & \text{for } MZ_{\min} \leq MZ \leq MZ_{\max} \end{cases}$$

with $F(I_{spectrum}(MZ))$ defined in such a way that:

$$\int\limits_{MZ_{min}}^{MZ_{max}} F\big(I_{spectrum}(MZ)\big)dMZ = 1 \quad F\big(I_{spectrum}(MZ_{min})\big)=0 \quad F\big(I_{spectrum}(MZ_{max})\big)=0$$

Finally the intensity score *IS* for a single-particle spectrum can then be defined as the integral of the product of the shape function and the single-particle ion intensity spectrum over the whole mass range of the mass spectrometer:

$$IS_{feature} = \int\limits_{MZ_{min\,MS}}^{MZ=MZ_{max\,MS}} S_{feature}(MZ)I_{single\,particle}(MZ)dMZ \ .$$

Since the ion intensity is recorded with a finite sampling rate, the above integral is replaced with a discrete sum:

$$\hat{IS}_{feature} = \sum_{i=i(MZ_{min\,MS})}^{i=i(MZ_{max\,MS})} S_{feature}(MZ_i)I_{single\,particle}(MZ_i)\Delta MZ_i \ .$$

Thus the intensity score can be represented by the inner product of two vectors:

$$IS_{feature} = \overline{S}_{feature} \cdot \overline{I}_{single\,particle} \ ,$$

where

$\overline{S}$feature = $S_{feature}(MZ_i))$ for $i = i(MZ_{max\,MS})$ to $i = i(MZ_{min\,MS})$
is a feature selection vector, and
$\overline{I}_{single\,particle} = I_{singel\,particle}(MZ_i))\Delta MZ_i$ for $i = i(MZ_{max\,MS})$ to $i = i(MZ_{min\,MS})$ single-particle spectrum energy vector.

**Estimating P(I|A)**

Generally, MALDI spectra of micro-organisms are characterized by a limited number of peaks, typically 10 to 50 in the mass region between 2000 Da and 20000 Da. These peaks indicate the presence of substances such as proteins, peptides, etc.

Some of these substances are characteristic for large groups of organisms (genus) some of them are characteristic for small groups (species) and some of them maybe even for a single organism (strain).

Given a reference collection of bacteria, with sufficiently characteristic peak patterns, a library of probability density functions can be constructed. This library will enable classification of an unknown particle containing a single bacterium in terms of a probability that characteristic substances are present in the particle.

The total collection of features that needs to be represented in the reference library is the union of all features for all organisms:

$$S_n^m \ \text{with} \ \begin{cases} m = 1...M_{isolates} \\ n = 1...N^m_{features} \end{cases}$$

(note that the number of features per isolate, depends on the organism in question)

When the full feature collection is transformed into a set of feature selection vectors $S_n^m$, these vectors can be transformed into a feature selection matrix,

$$\overline{\overline{S}} = \left( \left( \overline{S}_1^1 \middle| \overline{S}_2^1 \right) \quad \dots \quad \left( \overline{S}_1^m \right) \left( \overline{S}_2^m \right) \quad \dots \quad \left( \overline{S}_N^M \right) \right),$$

with NS columns, equal to the total number of features present feature collection and $M_{MZ}$ rows, equal to the number of time (mass) samples in the single-particle mass spectra.

Multiplying the single-particle spectrum energy vector with this feature selection matrix yields an intensity score IS vector that holds the intensity score for all features:

$$\overline{IS}_{\text{single particle}} = \overline{I}_{\text{single particle}} \cdot \overline{\overline{S}} .$$

As indicated above, by processing a sufficiently large set of single-particle spectra that result from micro-organisms originating from a single isolate, it is possible to create the probability-density functions. Using the above feature-selection matrix, the probability density functions $P^m{}_{1...NS}$ associated with a particular isolate $m$ can be determined in one go.

**Estimating P(I|¬A)**

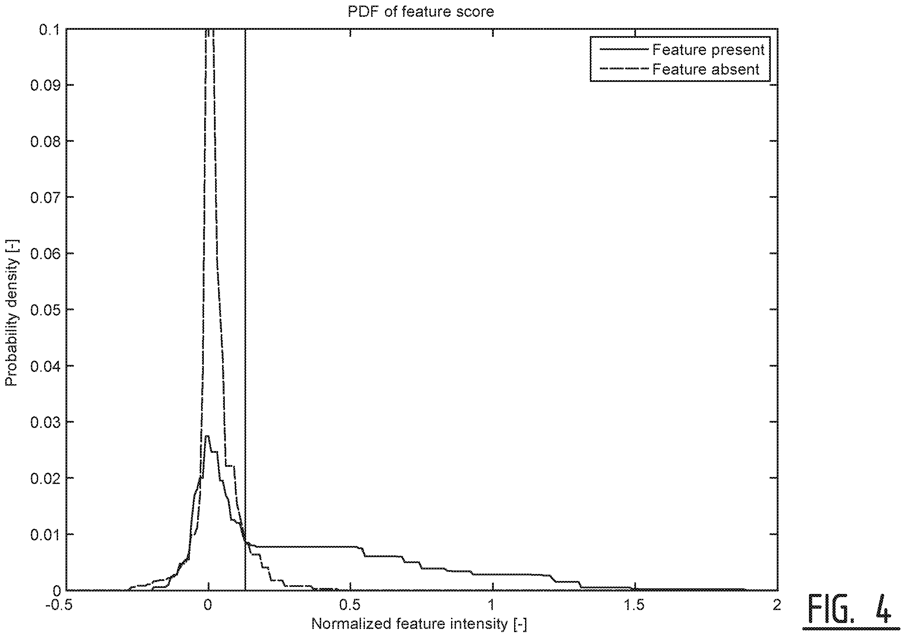

The probability density functions for the features that are related to a substance present in the organism under considerations will differ significantly from those that do not relate to substance present in the organism. As an example, figure 4 shows an example from the PDF of a feature for a particle collection containing the feature-inducing substance and a collection of particles that do not contain this substance.

Figure 4 shows that the amplitude of the PDF for the collection that contains the substance exceeds that of the collection that does not contain this substance for feature intensities above approximately 0.08 and vice versa.

In this simple example with only two possible particle types, one containing a substance $A^1$ that induces feature $Q_1$ and one containing substance $A^2$, the PDF for the collection that does not contain $A^1$, i.e. $\neg A^1$, is equal to the PDF of the particles that contain $A^2$:

$$P\left( I \mid \neg A^1 \right) = P\left( I \mid A^2 \right) .$$

As indicated above, given the probability density function $P(I/A)$ and $P(I/\neg A)$ it is possible to determine the probability that an individual particle contains the feature-inducing substance.

To calculate the probability that substance A is present, apart from the PDFs, the probability of encountering A, $P(A)$ must be provided. In this simple example, where only two types of particles are considered, particles that contain A and particles that do not contain A. Therefore, a probability of encountering A is assumed equal to $P(A) = P(\neg A) = 0.5$.

*Complex particle collections*

If more complex particle collections are considered, with $n$ possible particle types, where particle type $1$ contains substance $A^j$, the product $P(I/\neg A^j)P(\neg A^j)$ is equal to:

$$P\left( I \mid \neg A^j \right) P(\neg A^j) = \sum_k P\left( I \mid A^k \right) P(A^k) \text{ with } k \in \{1,2,3\dots,n\} \setminus j$$

Thus, as in the case of a binary mixture, in complex mixtures, the probability of encountering $P(A^j)$ is equal to $P(A^j)=1/n$.

**Analysis of a binary mixture**

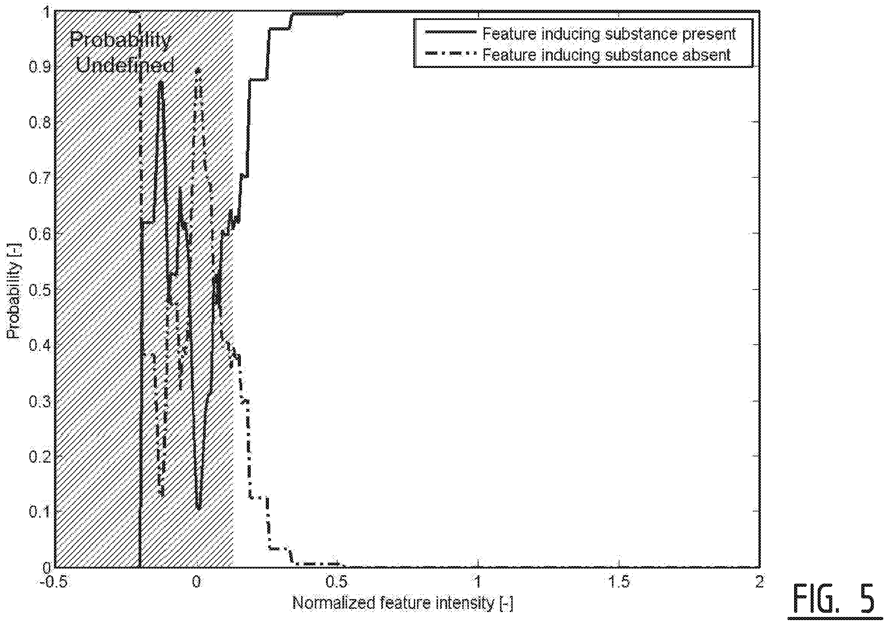

Based on the feature PDFs presented in figure 4 and Bayes' theorem the probability that feature-inducing substance A is present (or absent) as function of the (single-particle spectrum) feature intensity can be derived. Figure 5 shows this probability.

According to this figure above a critical intensity (indicated by the thin vertical line in figure 5 at approximately 0.08), the probability that the feature-inducing substance is present is consistently larger than the probability that this substance is absent.

Thus, above this intensity this feature is a reliable measure for the presence of this substance.

Below this intensity, the relation between the probability and the feature intensity is ambiguous. Hence, no judgement can be made with respect to the presence of the feature-inducing substance and $P(A/I)$ must be marked undefined.

To illustrate the functionality of the methodology described above, a (binary) mixture of pre-recorded single-particle spectra can be compiled. To unravel this mixture, for both particle types, features need to be defined and corresponding PDFs need to be estimated.

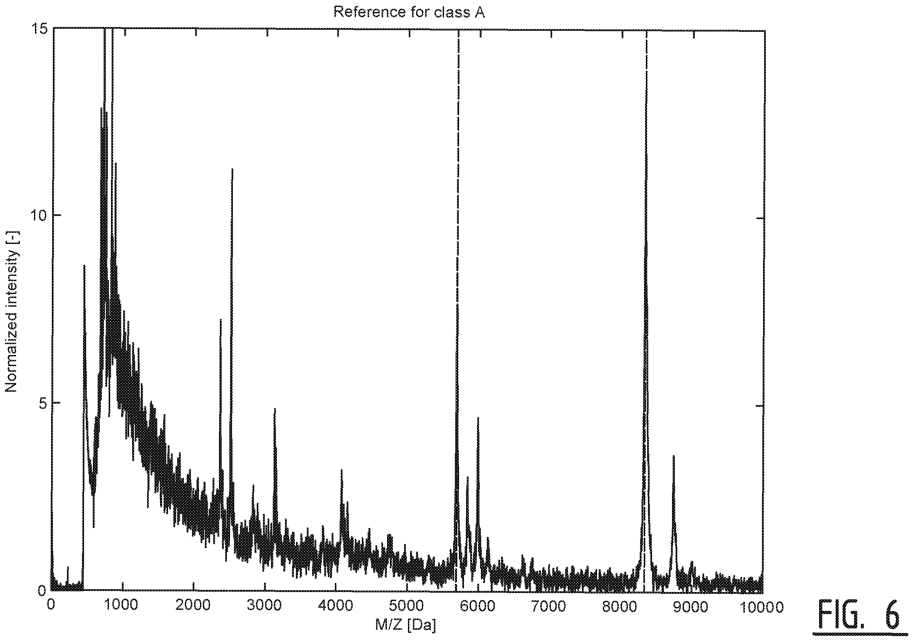

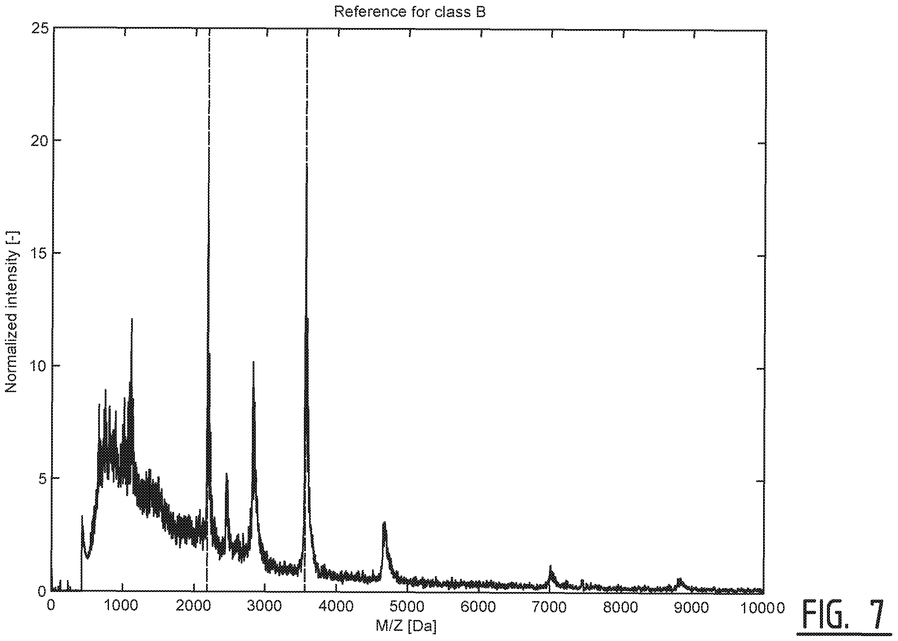

The figures 6 and 7 show the accumulated spectra of the particles used to estimate these feature PDFs. In this case, for both particle type two clear-cut peaks were selected as features (indicated by the vertical lines in figure 6 and figure 7).

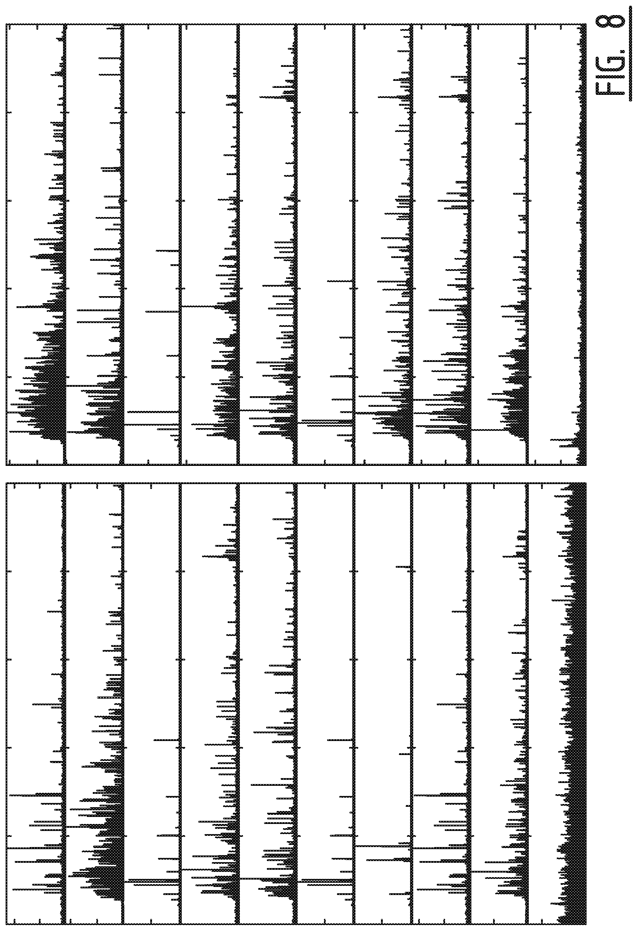

Figure 8 shows a sequence of single-particle spectra randomly selected from two batches of pre-recorded single-particle spectra (note that for the mixture, different batches are used than for estimating the PDFs). Again, Figure 8 illustrates the extreme variability in single-particle spectra and the apparent lack of correlation of the single-particle spectra with the reference spectra.

In this example the single-particle spectra are classified according to the following rules:

- the probability that a single-particle spectrum stems from one of the reference isolates, $P_{total}$, is derived from two single-feature probabilities for each of the two isolates according to the 'any feature present' rule as described above.
- only when the feature intensity $I$, exceeds the critical intensity for an unambiguous feature probability (see figure 5), a feature probability for this feature is allocated, otherwise the feature probability is rendered "undefined".
- The spectra are assigned to class (isolate) 1 when

$$P_{total}^1 > P_{total}^2 \wedge P_{total}^1 > P_{\min},$$

and to class 2 when

$$P_{total}^2 > P_{total}^1 \wedge P_{total}^2 > P_{\min},$$

wherein $P_{min}$ is a threshold probability that needs to exceeded in order to classify the spectrum. In this example a threshold probability $P_{min} = 0.9$ is used.

Those spectra that do not exceed the threshold probability, e.g. because both feature probabilities are rendered undefined, are assigned to the class "unknown".

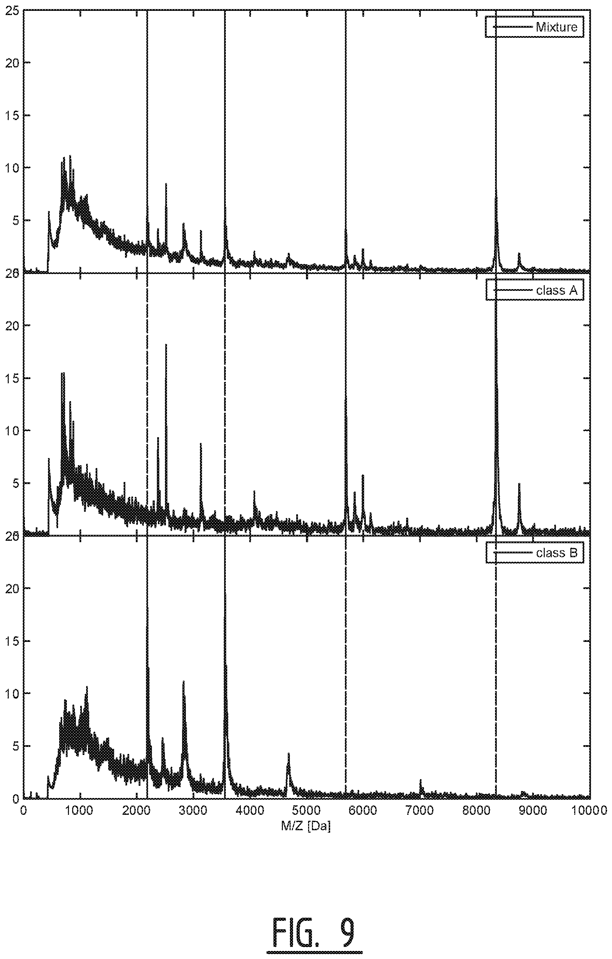

When the spectra that are assigned to one of the classes are accumulated, it appears that the resulting spectra for the two classes differ considerably from each other and from the accumulated spectrum of the mixture, see figure 9.

Inspection of figure 9 shows that at the peak locations (indicated by the vertical lines), the peaks are only retained for the spectrum corresponding to the reference isolate. This indicates that the classification algorithm is effective in the sense that it is able to select those spectra that contribute to a feature and is able to neglect those spectra that do not.

Therefore, the algorithm is capable of selecting single-particle spectra that contribute to a feature and that the accumulated spectra that result from a selection based on features that belong to different references differ significantly from each other. However, in some particular cases this will be no definitive proof that the two classes that are produced by the algorithm actually correspond to the references.

Therefore, as a final check a comparison can be made between the sample spectrum and the reference spectrum on the basis of other information than used for the classification.

### *Confirmation of the classification result*

To confirm that the single-particle spectra that are assigned to the different classes indeed correspond to the reference spectra, the accumulated single-particle spectra per class can be compared with the reference spectra.

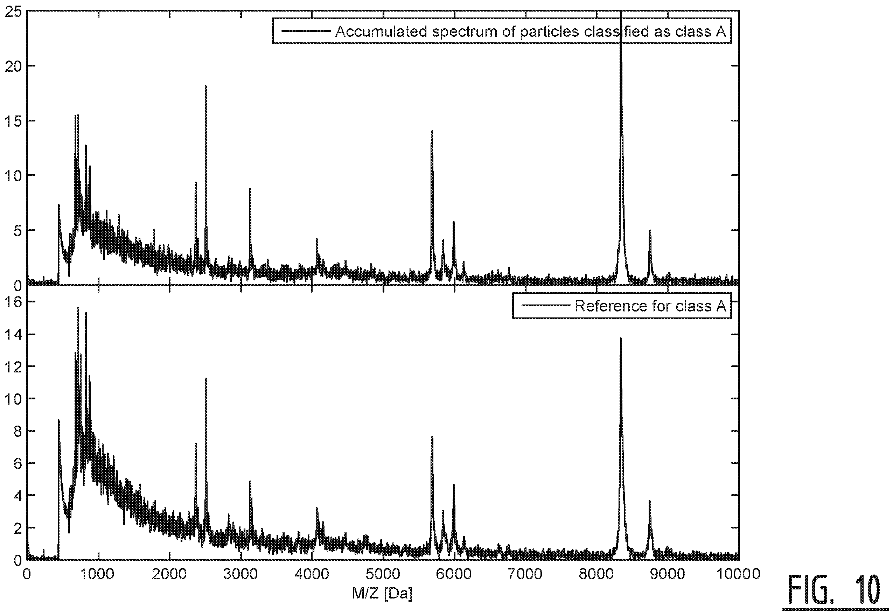

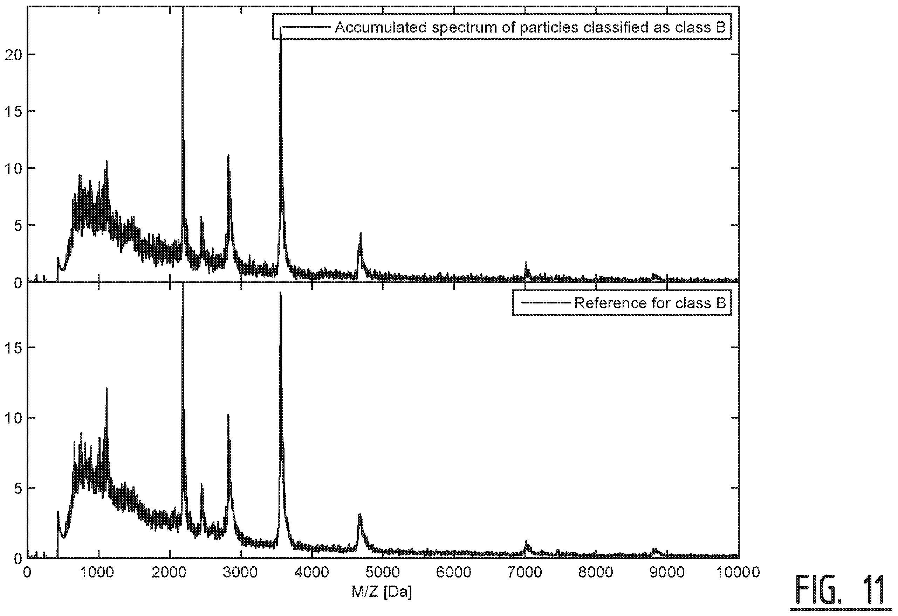

Figure 10 and figure 11 show this comparison for the two particles classes. These figures show that apart from the peaks used for the selection, also other the peaks present in the spectra that are compiled from the classified particles correspond to peaks in the reference spectra.

Furthermore, the spectra accumulated from the classified particles do not contain peaks that are not present in the reference spectra.

Both properties are a clear indication that the spectra compiled of classified spectra indeed correspond to the reference spectra. Consequently, the particles that resulted in the classified spectra indeed contain (fragments of) cells that correspond to the cells contained in the isolate used for the reference spectra.

*A hierarchical classification procedure*

Generally the accumulated spectra of bacteria contain somewhere between 10 to 40 distinguishable peaks. Potentially all peak locations (in terms of their mass) might be used as a feature for the single-particle classification.

Necessary condition for the suitability of a peak is that the probability-density function for the single-particle peak intensity differs sufficiently from the probability-density function for the other isolates in the reference library at the same mass.

A convenient measure for the difference/equality between two distributions is the Kolmogorov-Smirnov statistic. The Kolmogorov-Smirnov statistic quantifies a distance between two empirical cumulative distribution functions of two samples. If this statistic is sufficiently large the two underlying probability-density functions can be considered as distinguishable.

Owing to the finite resolution of the mass spectrometer, the more crowded the reference library, the larger the probability that peaks of different isolates (partly) overlap. Clearly, overlapping of peaks of different isolates renders them less suited for classification.

Hence, depending on the contents of the library of the reference library only a part of the peaks that are present in the spectra can effectively be used for single-particle classification.

The number classes that can be distinguished is determined by the number of peaks used. The number of classes that can be distinguished scales with $2^N$, where N is the number of non-overlapping peaks present in the reference library.

Initially, when all possible organisms must be considered, the reference library will be very crowded. Hence, the number of non-overlapping peaks will be low and the number of classes that can be distinguished will be low. Consequently, when analysing a sample containing organisms of mixed origin, the risk that a single class contains more than one species is relatively high.

However, when the single-particle spectra are stored individually, they can be classified a second time. Since now only the references need to be taken into account that belong to the class that these spectra were assigned to, a class-specific reference library can be constructed that is significantly less crowded than the original reference library.

Since the class-specific reference library is less crowded, the probability of overlap between peaks is reduced. Hence, a number of peaks not suited in the original library, will be suited in the class-specific library.

Consequently, the mixture containing sample can be discriminated further.

Thus by recursively classifying the mixture and redefining the reference library, a sample containing organisms of mixed origin can be discriminated to a large degree of specificity.

There is a link between the presence of peaks and the taxonomy. The choice of peaks can be motivated on the basis of the microbiology, through the use of peaks which are specific to the Order, Family, Genus, Species or Strain of the micro-organism. In this manner, a hierarchical classification scheme is realized.

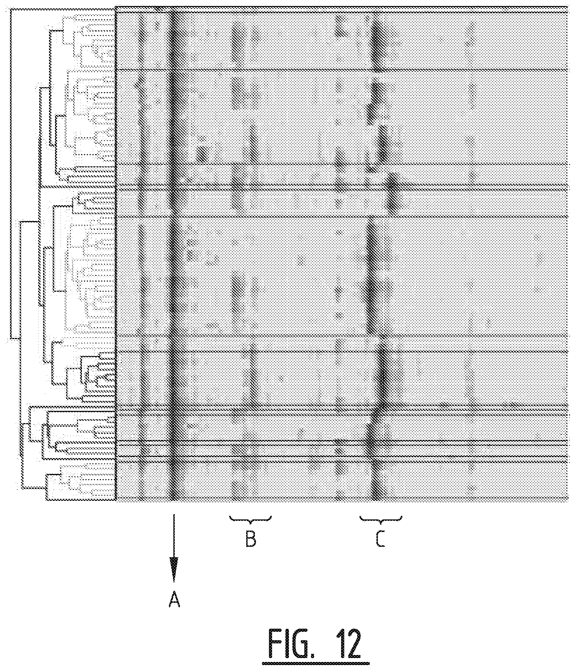

In figure 12 an example of a clustergram for a hierarchical classification scheme is shown. In the clustergram the peaks in the mass spectra of different strains of *Staphylococcus aureus* are depicted. The x-axis represents m/z. Different strains are plotted along the y-axis. The black areas correspond to peaks in the spectrum of the respective strains.

The clustergram shows that all the strains contain the species specific peaks A. However, they can be distinguished using strain specific peaks in the areas B and C.

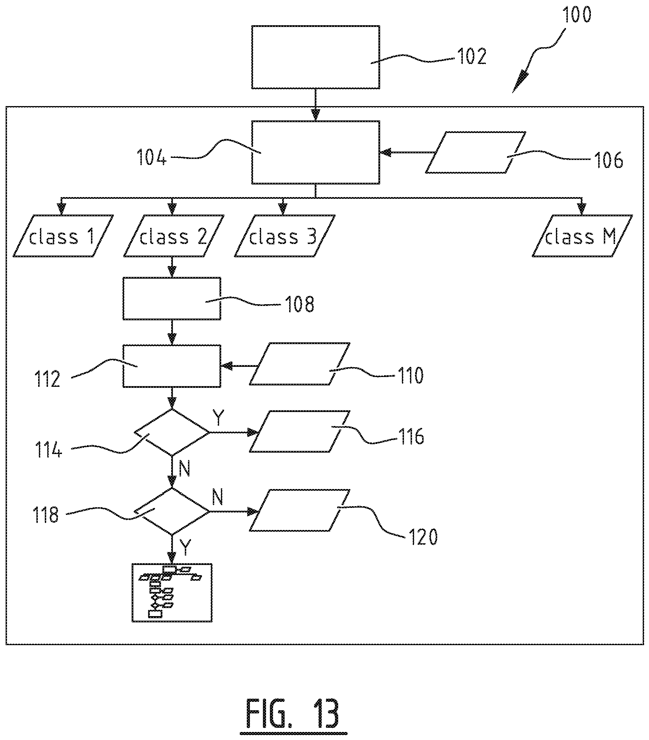

In an exemplary embodiment, a hierarchical classification scheme 100 (figure 13) according to the invention receives as input single particle spectra which are acquired in step 102. Subsequently the single particle spectra are classified in step 104 using a first reference set of PDFs 106. On the basis of this classification the sample spectra are classified in class 1, class 2, ..., class M.

For each group of sample spectra a comparison is made between the spectra in the group and the reference spectra of the respective class. This is illustrated for class 2. In a first step 108 the sample spectra are accumulated, i.e. a cumulative sample spectrum is created. In step 110, the cumulative reference spectrum is calculated. The cumulative sample spectrum and the cumulative reference spectrum are compared in step 112 by means of pattern matching.

In step 114 the match between the cumulative spectra is calculated. If the match is above a minimum threshold, the classification is considered correct and the algorithm return this classification in step 116. If the match is below the threshold, i.e. not acceptable, the algorithm checks if the cumulative sample spectrum is a mixed spectrum in step 118. For example, if additional peaks are present in the cumulative sample spectrum which are not present in the cumulative reference spectrum, the cumulative sample spectrum is considered to be a mixed spectrum.

When the spectrum is not a mixed spectrum, the algorithm returns this information and calculates the taxonomic distance of the spectrum to the closest related organism in step 120. In other words, the organism falls outside the classes of library 106. However, in a subsequent step, the spectra can be compared to a different library.

If in step 118 it is determined that the spectrum is a mixed spectrum, the algorithm returns to step 104, wherein a different library is used. This new library contains the subclasses of the class of the mixed spectrum, in this case the subclasses of class 2.

In other words, in case a mixed spectrum is observed, the algorithm goes deeper down the hierarchy by looking at subclasses of the respective class. When a match is found in 114 this procedure stops. If no match is found and no mixed spectrum is observed, the organism falls outside the original classes 106.

Below, the pseudo code for steps of the method according to the algorithm is shown.

**Generation and selection of probability density functions**

**%Generating the feature selection vectors%**

```
LOOP over isolates
    LOOP over single particle spectra files
        Read single particle spectrum as an intensity
    array
        Calculate total ion count
        Normalize the spectrum with the total ion count
        Add normalized spectrum to sum spectrum
    END loop
    Display summed spectrum
    Mark spectral features (either by hand or
    automatically)
    Store feature/isolate identification
    LOOP over features
        Extract feature-shape functions
        Store feature-shape function as feature-selection
    vector
    END loop
END loop
%%%%%%%%%%%%%%%%%%%%%%%%%%%%%%%%%%%%%%%%%%%%%%%%%%%%%
```

**%Generating the feature probability density functions%**

```
LOOP over isolates
    LOOP over single-particle files
        Read single particle spectrum as an intensity
    array
        Extract feature intensities for all features
        belonging to all isolates
        Store feature intensity for all features in
        corresponding feature-intensity arrays
    END loop
    LOOP over features
        Bin the elements of the feature-intensity arrays
        into appropriately spaced containers
        Return the number of elements per container in a
        feature-intensity frequency array
        Divide the feature intensity frequency array by the
        number of single-particle spectra processed
    END loop
END loop
%%%%%%%%%%%%%%%%%%%%%%%%%%%%%%%%%%%%%%%%%%%%%%%%%%%%%
```

**%Select feature probability density functions%**

```
LOOP over feature-probability density functions
      Generate cumulative probability density function
END loop
LOOP over isolates
      Select features 'native' for the isolate according to
      the feature/isolate identification table
          LOOP over 'native' features
            LOOP over 'other' features
              Determine and store the Kolmogorov-Smirnov
                statistic for each combination of native
                features and other features
            END loop

            Determine the smallest Kolmogorov-Smirnov
            statistic for each 'native' feature and store
            as minKS
          END loop
      Select the N (a number to be specified by the user
          but at least one) features with the largest
          minKS
      Store the feature selection for each isolate
END loop
```

*Mixture analysis*

**%Single-particle classification%**

```
WAIT loop
     Read single-particle mass spectrum
     Normalize single-particle mass spectrum
     Determine intensity of (selected) features
     LOOP over (selected) features
          LOOP over isolates
               Determine the P(I|A^m_n)P(A) and the
                    contribution to P(I|¬A_n)P(¬A)
          END loop
          Determine P(A^m_n|I)
     END loop
     LOOP over isolates
          Using the 'native' feature list, determine P_total
               for each isolate
     END Loop
     Assign the spectrum to one of the classes that
          correspond to the references or to the class
          unknown
END loop
```

```
LOOP over classes
    If number of spectra larger than minimum number
        Sum all spectra assigned to the class
        Smooth accumulated spectrum
        Resample smoothed spectrum
        Subtract baseline from resampled spectrum
        Extract peaks
        Classify peak list into sub classes (e.g. species)
            that belong to the class under consideration
            (e.g. genus)
        IF classification successful
            Report Identity
        ELSE
            Check whether mixture of species within class
            may be present
            IF mixture present
                Generate new feature set for further
                discrimination of the mixture
                Perform new single-particle classification
                for those particles that were assigned to the
                group under consideration
            ELSE
                Determine taxonomic distance of accumulated
                spectrum to reference spectra
            END
        END
    END
END loop
```

**Claims**

1.  Method for classification of micro-organisms of a biological sample comprising micro-organisms in one of at least two classes on the basis of spectral data comprising a MALDI MS spectrum or a MALDI TOF MS spectrum, the method comprising the steps of:

    creating a database for use in classification of a sample in one of at least two classes on the basis of spectral data, wherein the creating of the database comprises:

    a) obtaining at least two sets of first spectra for use as reference spectra, each set corresponding to a class of micro-organisms, wherein each set comprises spectra of reference samples of micro-organisms belonging to a same class wherein reference spectra are obtained from droplets each comprising a single micro-organism of said class;
    b) determining for each of the reference spectra the value of the same at least one quantity related to a spectral feature included in a selected one of said spectral data by applying a predetermined function or operation to each of the reference spectra; and

c) for each set of reference spectra, associating a probability to different values of said at least one quantity on the basis of the determined values, and constructing a probability density function from said quantity values and associated probabilities; and

wherein the classification of said micro-organisms comprises:

d) obtaining a spectrum from droplets each comprising a single micro-organism from the sample and determining the value of the same at least one quantity of this spectrum; and

e) calculating on the basis of the probability density functions obtained from the database and the value of the quantity for the obtained spectrum for each of said at least two classes a probability that the sample belongs to that class.

2. Method according to claim 1, wherein
the at least one quantity is selected on the basis of a characteristic spectral feature of a reference class.

3. Method according to any of the claims 1 or 2, comprising calculating, for a sample spectrum for which a value $I_i$ is obtained related to a spectral feature quantity $Q_i$, the probability $P(A^j/I_i)$ that the sample belongs to the reference class $A^j$ given the value $I_i$ according to:

$$P\left(A^j \middle| I_i\right) = \frac{P\left(I_i \middle| A^j\right)P(A^j)}{P(I_i|A^j)P(A^j) + \sum_{k \neq j} P(I_i|A^k)P(A^k)}$$

wherein $P(I_i|A^j)$ is the probability associated with the value $I_i$ for the reference class $A^j$, and $P(I_i|A^k)$ with $k \neq j$ is the probability associated with the value $I_i$ for at least one reference class different from the reference class $A^j$.

4. Method according to any of the claims 1-3, wherein step b) comprises determining for each of the reference spectra the value of the same at least two quantities related to a spectral feature and step e) comprises combining the probabilities obtained for all quantities to an overall probability that the sample belongs to the respective class.

5. Method according to any of the claims 1-4, wherein

the value relates to the intensity at at least one predetermined spectral value or within a predetermined range of spectral values, and/or
the value is normalized.

6. Method according to any of the claims 1-5, wherein the value is determined by multiplying the intensities of the respective spectrum over a predetermined range of spectral values with a weighting function, wherein preferably the weighting function is based on a cumulative spectrum of spectra of the same class.

7. Method according to any of the claims 1-6, wherein

the value relates to the ratio between the intensities at predetermined spectral values or within predetermined spectral ranges, or
the value relates to the location of a peak within a predetermined range of spectral values, and/or
the value is a vector.

8. Method according to any of the claims 1-7, wherein steps d) and e) are performed for a first set of reference classes and subsequently for a second set of reference classes, wherein the second set is selected on the basis of the classification of the sample in one of the reference classes of the first set, wherein:

the sample is a biological sample comprising micro-organisms and classification comprises the classification of micro-organisms; and
the first set and second set are selected on the basis of a biological classification hierarchy.

9. Method according to any of the claims 1-8, step d) comprising obtaining at least two spectra from the sample and determining the value of the same at least one quantity of these at least two spectra and step e) comprising combining the sample spectra classified in the same class into a cumulative spectrum and comparing this cumulative spectrum to a cumulative reference spectrum obtained by combining the reference spectra of the respective class.

10. Data storage medium comprising a computer program which, when executed on a computer, executes the steps of the method according to any of the preceding claims.

11. System for classifying a sample on the basis of spectral data, comprising:

- means arranged to obtain a spectrum from a single micro-organism from a sample;
- analyzing means arranged to perform the method according to any of the claims 1-9.

**Patentansprüche**

1. Verfahren zur Klassifikation von Mikroorganismen einer biologischen Probe, welche Mikroorganismen in einer von zumindest zwei Klassen umfasst, auf der Basis von Spektraldaten, welche ein MALDI MS Spektrum oder ein AMLDI TOF MS Spektrum umfassen, wobei das Verfahren die folgenden Schritte umfasst:

Erzeugen einer Datenbank zur Verwendung beim Klassifizieren einer Probe in eine von zumindest zwei Klassen auf der Basis von Spektraldaten, wobei das Erzeugen der Datenbank umfasst:

a) Erhalten von zumindest zwei Sätzen von ersten Spektren zur Verwendung als Referenzspektren, wobei jeder Satz einer Klasse von Mikroorganismen entspricht und wobei jeder Satz Spektren von Referenzproben von Mikroorganismen umfasst, welche zu einer gleichen Klasse gehören, wobei Referenzspektren aus Tröpfchen erhalten werden, welche jeweils einen einzigen Mikroorganismus aus der Klasse umfassen;
b) Bestimmen, für jedes von den Referenzspektren, den Wert von der gleichen zumindest einen Quantität bezogen auf ein spektrales Merkmal, welches in einem Ausgewählten aus den Spektraldaten enthalten ist durch Anwenden einer vorbestimmten Funktion oder Operation auf jedes von den Referenzspektren; und
c) Assoziieren, für jeden Satz von Referenzspektren, einer Wahrscheinlichkeit mit verschiedenen Werten von zumindest einer Quantität auf der Basis von den bestimmten Werten, und Bilden einer Wahrscheinlichkeitsdichtefunktion aus den Quantitätswerten und assoziierten Wahrscheinlichkeiten; und

wobei die Klassifikation von den Mikroorganismen umfasst:

d) Erhalten eines Spektrums aus Tröpfchen, welche jeweils einen einzigen Mikroorganismus aus der Probe umfassen, und Bestimmen des Werts von der gleichen zumindest einen Quantität aus diesem Spektrum; und
e) Berechnen, auf der Basis der Wahrscheinlichkeitsdichtefunktionen, welche aus der Datenbank und dem Wert der Quantität für das erhaltene Spektrum für jede von den zumindest zwei Klassen erhalten werden, einer Wahrscheinlichkeit, dass die Probe zu der Klasse gehört.

2. Verfahren gemäß Anspruch 1, wobei die zumindest eine Quantität ausgewählt wird auf der Basis von einem charakteristischen spektralen Merkmal einer Referenzklasse.

3. Verfahren gemäß irgendeinem der Ansprüche 1 oder 2, welches ein Berechnen, für ein Probenspektrum, für welches ein Wert $I_i$ erhalten wird, welcher auf eine Quantität eines spektralen Merkmals $Qi$ bezogen ist, der Wahrscheinlichkeit $P(A^i / I_i)$ umfasst, dass die Probe zu der Referenzklasse $A^i$ gehört mit gegebenem $I_i$ Wert gemäß:

$$P\left(A^j \middle| I_i\right) = \frac{P\left(I_i \middle| A^j\right) P\left(A^j\right)}{P\left(I_i \middle| A^j\right) P\left(A^j\right) + \sum_{k \neq j} P\left(I_i \middle| A^k\right) P\left(A^k\right)}$$

wobei $P(I_i / A^j)$ die Wahrscheinlichkeit ist, welche mit dem Wert $I_i$ für die Referenzklasse $A^j$ assoziiert ist, und $P(I_i / A^k)$ mit $k \neq j$ die Wahrscheinlichkeit ist, welche mit dem Wert $I_i$ für zumindest eine Referenzklasse assoziiert ist, welche unterschiedlich ist zu der Referenzklasse $A^j$.

**4.** Verfahren gemäß irgendeinem der Ansprüche 1 - 3, wobei der Schritt b) ein Bestimmen des Werts von den gleichen zumindest zwei Quantitäten bezogen auf ein spektrales Merkmal für jedes der Referenzspektren umfasst, und der Schritt e) ein Kombinieren der Wahrscheinlichkeit umfasst, welche für alle Quantitäten erhalten wurden, mit einer Gesamtwahrscheinlichkeit, dass die Probe zu der jeweiligen Klasse gehört.

**5.** Verfahren gemäß irgendeinem der Ansprüche 1 - 4, wobei sich der Wert auf die Intensität an zumindest einem vorbestimmten spektralen Wert oder innerhalb eines vorbestimmten Bereichs von Spektralwerten bezieht, und/oder der Wert normiert ist.

**6.** Verfahren gemäß irgendeinem der Ansprüche 1 - 5, wobei der Wert bestimmt wird durch Multiplizieren der Intensitäten von dem jeweiligen Spektrum über einen vorbestimmten Bereich von Spektralwerten mit einer Gewichtungsfunktion, wobei vorzugsweise die Gewichtungsfunktion auf einem kumulativen Spektrum von Spektren von der gleichen Klasse basiert.

**7.** Verfahren gemäß irgendeinem der Ansprüche 1 - 6, wobei sich der Wert auf das Verhältnis zwischen den Intensitäten bei vorbestimmten Spektralwerten oder innerhalb vorbestimmter Spektralbereiche bezieht, oder wobei sich der Wert auf die Stelle von einem Peak innerhalb eines vorbestimmten Bereichs von Spektralwerten bezieht, und/oder der Wert ein Vektor ist.

**8.** Verfahren gemäß irgendeinem der Ansprüche 1 - 7, wobei die Schritte d) und e) für einen ersten Satz von Referenzklassen durchgeführt werden und anschließend für einen zweiten Satz von Referenzklassen, wobei der zweite Satz ausgewählt wird auf der Basis von der Klassifikation von der Probe in einer von den Referenzklassen von dem ersten Satz, wobei:

die Probe eine biologische Probe ist, welche Mikroorganismen umfasst, und die Klassifikation eine Klassifikation von Mikroorganismen umfasst; und der erste Satz und der zweite Satz auf der Basis einer biologischen Klassifikationshierarchie ausgewählt werden.

**9.** Verfahren gemäß irgendeinem der Ansprüche 1 - 8, wobei der Schritt d) ein Erhalten von zumindest zwei Spektren aus der Probe umfasst, und ein Bestimmen des Werts von der gleichen zumindest einen Quantität von diesen zumindest zwei Spektren, und wobei der Schritt e) ein Kombinieren der Spektren der Probe, welche in der gleichen Klasse in einem kumulativen Spektrum klassifiziert wurden, und Vergleichen dieses kumulativen Spektrums mit einem kumulativen Referenzspektrum, welches durch Kombinieren der Referenzspektren von der jeweiligen Klasse erhalten wird.

**10.** Datenspeichermedium, welches ein Computerprogramm umfasst, welches, wenn auf einem Computer ausgeführt, die Schritte des Verfahrens gemäß irgendeinem der vorherigen Ansprüche ausführt.

**11.** System zum Klassifizieren einer Probe auf der Basis von Spektraldaten, umfassend:

- Mittel, welche angeordnet sind, um ein Spektrum aus einem einzigen Mikroorganismus von einer Probe zu erhalten;
- Analysemittel, welche angeordnet sind, um das Verfahren gemäß irgendeinem der Ansprüche 1 - 9 durchzuführen.

**Revendications**

**1.** Procédé de classification de micro-organismes d'un échantillon biologique comprenant des micro-organismes dans l'une parmi au moins deux classes sur la base de données spectrales comprenant un spectre en spectrométrie de masse à désorption-ionisation par impact laser assistée par matrice, MALDI MS, ou un spectre en MALDI MS à temps de vol, MALDI TOF MS, le procédé comprenant les étapes de :

la création d'une base de données pour une utilisation dans la classification d'un échantillon dans l'une parmi au moins deux classes sur la base de données spectrales, dans lequel la création de la base de données comprend :

a) l'obtention d'au moins deux ensembles de premiers spectres pour une utilisation comme spectres de référence, chaque ensemble correspondant à une classe de micro-organismes, dans lequel chaque ensemble comprend des spectres d'échantillons de référence de micro-organismes appartenant à une même classe, dans lequel les spectres de référence sont obtenus à partir de gouttelettes comprenant chacune un unique micro-organisme de ladite classe ;

b) la détermination, pour chacun des spectres de référence, de la valeur de la même au moins une quantité se rapportant à une particularité spectrale incluse dans une donnée sélectionnée desdites données spectrales par l'application d'une fonction ou d'une opération prédéterminée sur chacun des spectres de référence ; et

c) pour chaque ensemble de spectres de référence, l'association d'une probabilité avec différentes valeurs de ladite au moins une quantité sur la base des valeurs déterminées, et la construction d'une fonction de densité de probabilité à partir desdites valeurs de quantité et des probabilités associées ; et

dans lequel la classification desdits micro-organismes comprend :

d) l'obtention d'un spectre à partir de gouttelettes comprenant chacune un unique micro-organisme à partir de l'échantillon et la détermination de la valeur de la même au moins une quantité de ce spectre ; et

e) le calcul, sur la base des fonctions de densité de probabilité obtenues à partir de la base de données et de la valeur de la quantité pour le spectre obtenu pour chacune desdites

au moins deux classes, d'une probabilité que l'échantillon appartienne à cette classe.

2. Procédé selon la revendication 1, dans lequel
l'au moins une quantité est sélectionnée sur la base d'une particularité spectrale caractéristique d'une classe de référence.

3. Procédé selon l'une quelconque des revendications 1 ou 2, comprenant le calcul, pour un spectre d'échantillon pour lequel une valeur $I_i$ est obtenue se rapportant à une quantité de particularité spectrale $Q_i$, la probabilité $P(A^j/I_i)$ que l'échantillon appartienne à la classe de référence $A^j$ étant donnée la valeur $I_i$ selon :

$$P\left(A^j\middle|I_i\right) = \frac{P\left(I_i\middle|A^j\right)P\left(A^j\right)}{P\left(I_i\middle|A^j\right)P\left(A^j\right) + \sum_{k \neq j} P\left(I_i\middle|A^k\right)P\left(A^k\right)}$$

dans lequel $P(I_i|A^j)$ est la probabilité associée avec la valeur $I_i$ pour la classe de référence $A^j$, et $P(I_i|A^k)$ avec $k \# j$ est la probabilité associée avec la valeur $I_i$ pour au moins une classe de référence différente de la classe de référence $A^j$.

4. Procédé selon l'une quelconque des revendications 1 à 3, dans lequel l'étape b) comprend la détermination, pour chacun des spectres de référence, de la valeur des mêmes au moins deux quantités se rapportant à une particularité spectrale et l'étape e) comprend la combinaison des probabilités obtenues pour toutes les quantités en une probabilité globale que l'échantillon appartienne à la classe respective.

5. Procédé selon l'une quelconque des revendications 1 à 4, dans lequel
la valeur se rapporte à l'intensité à au moins une valeur spectrale prédéterminée ou à l'intérieur d'une plage prédéterminée de valeurs spectrales, et/ou la valeur est normalisée.

6. Procédé selon l'une quelconque des revendications 1 à 5, dans lequel la valeur est déterminée par la multiplication des intensités du spectre respectif sur une plage prédéterminée de valeurs spectrales avec une fonction de pondération, dans lequel de préférence la fonction de pondération est basée sur un spectre cumulatif de spectres de la même classe.

7. Procédé selon l'une quelconque des revendications 1 à 6, dans lequel
la valeur se rapporte au rapport entre les intensités à des valeurs spectrales prédéterminées ou à l'intérieur de plages spectrales prédéterminées, ou
la valeur se rapporte à l'emplacement d'un pic à l'intérieur d'une plage prédéterminée de valeurs spectrales, et/ou la valeur est un vecteur.

8. Procédé selon l'une quelconque des revendications 1 à 7, dans lequel les étapes d) et e) sont réalisées pour un premier ensemble de classes de référence et ultérieurement pour un second ensemble de classes de référence, dans lequel le second ensemble est sélectionné sur la base de la classification de l'échantillon dans l'une des classes de référence du premier ensemble, dans lequel :

l'échantillon est un échantillon biologique comprenant des micro-organismes et la classification comprend la classification des micro-organismes ; et
le premier ensemble et le second ensemble sont sélectionnés sur la base d'une hiérarchie de classification biologique.

9. Procédé selon l'une quelconque des revendications 1 à 8, l'étape d) comprenant l'obtention d'au moins deux spectres à partir de l'échantillon et la détermination de la valeur de la même au moins une quantité de ces au moins deux spectres et l'étape e) comprenant la combinaison des spectres d'échantillons classés dans la même classe en un spectre cumulatif et la comparaison de ce spectre cumulatif avec un spectre de référence cumulatif obtenu par la combinaison des spectres de référence de la classe respective.

10. Support de stockage de données comprenant un programme d'ordinateur qui, lorsqu'il est exécuté sur un ordinateur, exécute les étapes du procédé selon l'une quelconque des revendications précédentes.

11. Système de classification d'un échantillon sur la base de données spectrales, comprenant :

- des moyens conçus pour obtenir un spectre à partir d'un unique micro-organisme à partir d'un échantillon ;
- des moyens d'analyse conçus pour réaliser le procédé selon l'une quelconque des revendications 1 à 9.

FIG. 1

FIG. 2

FIG. 3

PDF of feature score

FIG. 4

FIG. 5

Reference for class A

FIG. 6

EP 2 836 958 B1

FIG. 7

FIG. 8

FIG. 9

FIG. 10

EP 2 836 958 B1

FIG. 11

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070184455 A1

- WO 2010021548 A